# EUROPÄISCHE PATENTANMELDUNG

(11) **EP 4 817 493 A1**
(43) Veröffentlichungstag der Anmeldung: **07.10.2026**
(21) Anmeldenummer: 26161623.9
(22) Anmeldetag: 02.03.2026
(51) Int. Cl.: A47L 11/40

(54) **BASISSTATION FÜR EINEN BODENREINIGER**

(30) Priorität: 31.03.2025 DE 102025112505
(71) Anmelder: BSH Hausgeräte GmbH, 81739 München (DE)
(72) Erfinder: Fremerey, Maximilian, 97633 Saal (DE); Sadhanala, Hari Teja, 97616 Bad Neustadt (DE)

(57) **Zusammenfassung**

Die Erfindung betrifft ein Verfahren (200) zur Reinigung und/oder Desinfektion eines Wischtextils (109) eines Reinigungsgeräts (103) in einer Reinigungs- und/oder Desinfektionsstation (106). Das Verfahren (200) umfasst die Schritte:
a) Pulsierendes Zuführen einer definierten Menge einer Reinigungs- oder Desinfektionsflüssigkeit auf das Wischtextil (109) für eine vorgegebene erste Zeitdauer,
b) Bewegen des Wischtextils (109) über mechanische Strukturen (136), um Schmutz sowie überschüssige Flüssigkeit aus dem Wischtextil (109) auszupressen und in eine Auffangeinrichtung (148) abzuleiten, und
c) Stoppen der Flüssigkeitszufuhr und Fortsetzen der Bewegung des Wischtextils (109) über die mechanischen Strukturen (136) für eine vorgegebene zweite Zeitdauer, wobei die erste Zeitdauer jeweils kürzer ist als die nachfolgende zweite Zeitdauer. Die Erfindung betrifft ferner Reinigungs- und/oder Desinfektionsstation (106) für ein Reinigungs- und/oder Desinfektionssystem (100) zur Reinigung und/oder Desinfektion eines Wischtextils (109) eines Reinigungsgeräts (103). Außerdem betrifft die Erfindung ein Reinigungs- und/oder Desinfektionssystem (100), umfassend eine solche Reinigungs- und/oder Desinfektionsstation (106).

## Beschreibung

Die vorliegende Erfindung betrifft ein Verfahren zur Reinigung und/oder Desinfektion eines Wischtextils eines Reinigungsgeräts in einer Reinigungs- und/oder Desinfektionsstation sowie eine Reinigungs- und/oder Desinfektionsstation für ein entsprechendes System.

Derzeit bekannte Reinigungsroboter können sowohl staubsaugen als auch nass wischen und verwenden meist flache Wischtextilien aus natürlichen und/oder synthetischen Fasern. Diese Wischtextilien nehmen zunächst Wasser und beim Reinigen dann Schmutz vom Boden auf. Um den Reinigungseffekt zu verbessern, sind viele dieser Roboter mit einem Aktor ausgestattet, der das Wischtextil in Rotation oder Oszillation versetzt. Nach einer bestimmten Einsatzzeit sättigt sich das Wischtextil jedoch mit Schmutz, wodurch es nicht mehr reinigt, sondern den Schmutz verteilt. Moderne Reinigungsroboter verfügen daher über eine Basisstation mit einer Reinigungs- und/oder Desinfektionsfunktion, die eine automatische Reinigung und gegebenenfalls eine Desinfektion des Wischtextils ermöglicht. In der Basisstation wird das Wischtextil durch mechanische Strukturen in Kombination mit einer Flüssigkeitszufuhr behandelt.

Ein ähnliches Verfahren ist aus der CN 1 17 694 779 A bekannt. Dort wird ein Reinigungsteil zunächst mit Flüssigkeit versorgt, anschließend wird die Flüssigkeitszufuhr gestoppt, und das Reinigungsteil bewegt sich weiter, während ein Abstreifmechanismus den Schmutz entfernt. Die verunreinigte Flüssigkeit wird durch ein Unterdrucksystem in einen Sammelbehälter überführt und abschließend abgesaugt. Dieses Verfahren reduziert den Wasserverbrauch und erhöht die Reinigungseffizienz, berücksichtigt jedoch keine gezielte Steuerung der Flüssigkeitszufuhr oder eine mechanische Schmutzauspressung.

Eine der vorliegenden Erfindung zu Grunde liegende Aufgabe besteht darin, ein verbessertes Verfahren sowie eine Vorrichtung bereitzustellen, die eine effiziente Reinigung und/oder Desinfektion eines Wischtextils ermöglichen und den Flüssigkeitsverbrauch minimieren. Dabei soll insbesondere die Flüssigkeitsmenge reduziert und gleichzeitig die Reinigungseffizienz maximiert werden. Die Erfindung löst diese Aufgabe mittels der Gegenstände der unabhängigen Ansprüche. Unteransprüche geben bevorzugte Ausführungsformen wieder.

Ein Verfahren zur Reinigung und/oder Desinfektion eines Wischtextils eines Reinigungsgeräts in einer Reinigungs- und/oder Desinfektionsstation, umfassend die folgenden Schritte:
a) Pulsierendes Zuführen einer definierten Menge einer Reinigungs- oder Desinfektionsflüssigkeit auf das Wischtextil für eine vorgegebene erste Zeitdauer,
b) Bewegen des Wischtextils über mechanische Strukturen, um Schmutz sowie überschüssige Flüssigkeit aus dem Wischtextil auszupressen und in eine Auffangeinrichtung abzuleiten, und
c) Stoppen der Flüssigkeitszufuhr und Fortsetzen der Bewegung des Wischtextils über die mechanischen Strukturen für eine vorgegebene zweite Zeitdauer, wobei die erste Zeitdauer jeweils kürzer ist als die nachfolgende zweite Zeitdauer.

Die Flüssigkeitszufuhr erfolgt über eine Flüssigkeitszuführeinrichtung, die als Membran- oder Peristaltikpumpe ausgeführt sein kann. Alternativ kann eine Drucksteuerung über ein Ventilsystem verwendet werden, um die Flüssigkeitsmenge genauer zu dosieren. Die Flüssigkeit wird aus einem Reservoir angesaugt und auf das Wischtextil gefördert.

Die Auffangeinrichtung ist beispielsweise eine wasserdichte Wanne oder ein Behälter, dessen Innenwandung in Bezug auf eine Horizontale geneigt ausgeführt ist, um Flüssigkeit gezielt an einer tiefsten Stelle zu sammeln. Beispielsweise ist die Innenwandung, insbesondere der Wannengrund, um mindestens eine Achse geneigt.

In Schritt c) wird das Wischtextil nach Beendigung der Flüssigkeitszufuhr weiter ausgepresst, um eine maximale Entwässerung zu erzielen. Durch den wiederholten Kontakt mit den mechanischen Strukturen wird Schmutz aus dem Textil entfernt und überschüssige Flüssigkeit in die Auffangeinrichtung geleitet.

"Pulsierende Zuführung" meint die intermittierende Flüssigkeitsapplikation in festgelegten Intervallen, um eine ressourcenschonende Befeuchtung zu erzielen. "Definierte Menge" bezeichnet die exakt dosierte Flüssigkeitsmenge, die an den Verschmutzungsgrad und die zu reinigende Fläche angepasst werden kann.

Die Reinigungs- oder Desinfektionsflüssigkeit ist eine Flüssigkeit, die zur Entfernung von Schmutz oder zur Reduzierung von Keimen auf dem Wischtextil dient. Sie kann aus Wasser, einer Mischung aus Wasser und Reinigungsmittel (Reinigungsflüssigkeit) oder aus einer Mischung aus Wasser und Desinfektionsmittel (Desinfektionsflüssigkeit), beispielsweise Natriumhypochlorit, bestehen.

Das Wischtextil ist das Reinigungselement des Reinigungsgeräts, das während des Reinigungsvorgangs Schmutz und Flüssigkeit aufnimmt. Es kann aus synthetischen oder natürlichen Fasern bestehen und ist entweder als flaches Wischtuch oder als Wischmopp ausgeführt. Das Reinigungsgerät kann mehrere solcher Wischtextile aufweisen, die mit dem hierin beschriebenen Verfahren gereinigt werden können. Das Wischtextil kann um eine im Wesentlichen vertikale Achse, also die z-Achse, rotieren oder oszillieren.

Die erste Zeitdauer umfasst die Flüssigkeitszufuhr und den Reinigungsvorgang, während die zweite Zeitdauer den Auspressvorgang beschreibt. Experimente haben gezeigt, dass die zweite Zeitdauer mindestens das Fünffache der ersten Zeitdauer betragen sollte, um eine effiziente Entwässerung zu gewährleisten. In diesem Sinn beträgt die zweite Zeitdauer bevorzugt mindestens das Fünffache der ersten Zeitdauer.

Eine mechanische Struktur ist eine feste oder bewegliche Komponente innerhalb eines Abreinigungsbereichs der Reinigungsstation, die in Kontakt mit dem Wischtextil tritt und mechanische Einwirkungen auf dieses ausübt. Sie kann als feststehender oder beweglicher Vorsprung mit dreieckigem Querschnitt ausgeführt sein. Sie kann beispielsweise als Vorsprung mit dreieckigem Querschnitt ausgeführt sein, um Schmutz und überschüssige Flüssigkeit aus dem Wischtextil zu entfernen.

Der Abreinigungsbereich ist der Abschnitt der Reinigungs- und/oder Desinfektionsstation, in dem das Wischtextil mit Reinigungs- oder Desinfektionsflüssigkeit behandelt sowie mechanisch ausgepresst wird. Der Abreinigungsbereich ist zur Aufnahme des Wischtextils eingerichtet, wobei die mechanischen Strukturen im Abreinigungsbereich vorgesehen sind.

Unter "Bewegen des Wischtextils" ist der Vorgang zu verstehen, bei dem das Wischtextil während der Reinigung über die mechanischen Strukturen geführt wird. Dies kann durch eine Bewegung des gesamten Reinigungsgeräts oder durch eine eigenständige Rotation oder Oszillation des Wischtextils erfolgen. In diesem Sinn wird das Wischtextil während Schritt b) und/oder Schritt c) rotierend oder oszillierend bewegt. Beim Bewegen des Wischtextils wird durch die mechanischen Strukturen Flüssigkeit aus dem Wischtextil ausgepresst. Es erfolgt also eine mechanische Beanspruchung des Wischtextils, beispielsweise durch Druck oder Reibung, so dass Flüssigkeit aus dem Wischtextil entfernt wird. Zur Bewegung des Wischtextils kann ein Aktuator des Reinigungsgeräts genutzt werden.

Die rotierende Bewegung beschreibt die kontinuierliche Drehbewegung des Wischtextils um eine zentrale Achse. Diese Bewegung dient dazu, die Reinigungseffizienz zu erhöhen, indem das Textil gleichmäßig über die zu reinigende Fläche geführt wird. Die oszillierende Bewegung ist eine hin- und hergehende Bewegung des Wischtextils entlang einer Achse. Sie unterstützt das Ablösen von festsitzendem Schmutz, indem sie das Textil wiederholt über die Bodenfläche oder mechanische Strukturen bewegt. Diese Bewegungsarten können ebenso vorteilhaft zur Reinigung bzw. Desinfektion des Wischtextils im genannten Verfahren eingesetzt werden.

Die überschüssige Flüssigkeit sowie die Schmutzartikel gelangen während dieses Vorgangs in die Auffangeinrichtung. Die Auffangeinrichtung nimmt die ausgepresste Flüssigkeit sowie die Schmutzartikel auf. Sie ist bevorzugt wasserdicht und kann geneigt ausgeführt sein, um die gesammelte Flüssigkeit kontrolliert in einen Ablauf oder eine vorbestimmte Sammelstelle zu leiten. Der Ablauf ist bevorzugt an der tiefsten Stelle der Auffangeinrichtung angeordnet, um Flüssigkeit durch Schwerkraft effizient abzuführen.

Vorzugsweise wird während Schritt a), Schritt b) und/oder Schritt c) die in der Auffangeinrichtung gesammelte Flüssigkeit über eine Flüssigkeitsabführeinrichtung, beispielsweise eine Pumpe oder ein Unterdrucksystem, in einen Abwassersammelbehälter abgeleitet wird. Der Abwassersammelbehälter ist ein, insbesondere geschlossener, Tank oder Behälter, in dem die aus dem Wischtextil entfernte Schmutzflüssigkeit gesammelt wird. Er verhindert das unkontrollierte Austreten von Flüssigkeit.

Nach einer besonderen Ausführungsform wird ein Verschmutzungsgrad der abgeleiteten Flüssigkeit erfasst. Der Verschmutzungsgrad beschreibt die Menge und Konzentration von Schmutzpartikeln oder anderen Verunreinigungen in der aufgefangenen Flüssigkeit. Der Verschmutzungsgrad der in den Abwassersammelbehälter abgeleiteten Flüssigkeit kann durch eine optische Trübungsmessung oder eine Leitfähigkeitsmessung erfasst werden.

Die Schritte a) bis c) können so oft wiederholt werden, bis der erfasste Verschmutzungsgrad einen Grenzwert unterschreitet. Die Anzahl oder Intensität der Pulszyklen kann entsprechend angepasst werden. In diesem Sinn kann die Pulsfrequenz der Flüssigkeitszufuhr dynamisch an den Verschmutzungsgrad angepasst werden. Der Grenzwert des Verschmutzungsgrades ist ein definierter Schwellenwert, der bestimmt, ab welchem Punkt die Reinigungszyklen fortgesetzt oder beendet werden. Erst wenn die gemessene Verschmutzung unter diesen Grenzwert fällt, wird der Reinigungsvorgang abgeschlossen. Ferner können mit jedem weiteren Reinigungszyklus die beiden Zeitdauern angepasst werden. Außerdem kann die eingesetzte Flüssigkeitsmenge während jeder Flüssigkeitszufuhr eingestellt und variiert werden.

Eine vorgeschlagenes Reinigungs- und/oder Desinfektionsstation für ein Reinigungs- und/oder Desinfektionssystem umfasst eine Flüssigkeitszuführeinrichtung zum pulsierenden Zuführen einer definierten Menge Reinigungs- oder Desinfektionsflüssigkeit auf das Wischtextil, eine Auffangeinrichtung zum Auffangen von Flüssigkeit, und einen Abreinigungsbereich mit mechanischen Strukturen, die dazu ausgebildet sind, durch mechanische Bearbeitung des Wischtextils Schmutz sowie überschüssige Flüssigkeit auszupressen und in die Auffangeinrichtung abzuleiten. Die mechanische Bearbeitung wird durch Bewegen des Wischtextils über die mechanischen Strukturen bewegt, so dass Schmutz sowie überschüssige Flüssigkeit bei Kontaktierung mit den mechanischen Strukturen ausgepresst wird und in die Auffangeinrichtung abfließt.

Die Reinigungs- und/oder Desinfektionsstation ist die Basisstation eines Reinigungs- oder Desinfektionssystems, in der das Wischtextil nach einem Reinigungsvorgang gereinigt und/oder desinfiziert wird. Sie kann zusätzliche Funktionen wie das automatische Nachfüllen von Flüssigkeiten oder das Entleeren von Schmutzwassertanks umfassen.

Die Flüssigkeitszuführeinrichtung ist ein Bauteil der Reinigungsstation, das die Reinigungs- oder Desinfektionsflüssigkeit aus einem Reservoir ansaugt und in pulsierender Weise auf das Wischtextil fördert. Sie kann als Pumpe, beispielsweise eine Membran- oder Peristaltikpumpe, ausgeführt sein und wird durch eine Steuerungseinheit pulsierend geregelt.

Vorzugsweise weist die Auffangeinrichtung einen Ablauf zum Ableiten der aufgefangenen Flüssigkeit in den Abwassersammelbehälter auf. Der Ablauf ist bevorzugt mit einem Abwassersammelbehälter fluidisch verbunden, wobei eine Flüssigkeitsabführeinrichtung vorgesehen und dazu ausgebildet ist, in der Auffangeinrichtung gesammelte Flüssigkeit über den Ablauf in den Abwassersammelbehälter abzuführen. Die Flüssigkeitsabführeinrichtung ist eine Pumpe oder ein Unterdrucksystem, das die gesammelte Schmutzflüssigkeit aus der Auffangeinrichtung in einen geschlossenen Abwassersammelbehälter transportiert. Alternativ kann ein Schwerkraftablauf mit ergänzender Pumpensteuerung verwendet werden, um eine besonders energiesparende Ableitung zu gewährleisten.

Ferner bevorzugt ist der Abwassersammelbehälter als geschlossenes System ausgebildet, um das Entweichen von Flüssigkeitsdämpfen zu verhindern. Das Entweichen von Flüssigkeitsdämpfen bezeichnet die unkontrollierte Verdunstung von Flüssigkeitsbestandteilen, insbesondere von Reinigungs- oder Desinfektionsflüssigkeiten.

Nach einem Ausführungsbeispiel sind die mechanischen Strukturen als Vorsprünge mit einer im Wesentlichen dreieckigen Querschnittsform ausgebildet, wobei die Vorsprünge mit dem Wischtextil in Kontakt kommen. Die Vorsprünge mit dreieckigem Querschnitt pressen das Wischtextil bei Bewegung aus, wobei die Flanken der Vorsprünge die ausgepresste Flüssigkeit in die Auffangeinrichtung ableiten. Die Vorsprünge sind insbesondere spitz zulaufend ausgeführt. Die mechanischen Strukturen können feststehend oder beweglich gelagert sein, um eine optimierte Anpassung an verschiedene Wischtextilien zu ermöglichen. Denkbar ist auch eine von einer dreieckigen Querschnittsform verschiedene Ausführung der mechanischen Strukturen. Die Form und Geometrie der mechanischen Strukturen kann an die Bauraumgegebenheiten und die Ausgestaltung des Wischtextils angepasst werden.

Mindestens eine der mechanischen Strukturen, insbesondere wenigstens einer der genannten Vorsprünge, weist eine oder mehrere Öffnungen zur Flüssigkeitszufuhr auf, wobei über die Öffnung oder die Öffnungen Wasser, Reinigungsflüssigkeit oder Desinfektionsflüssigkeit zugeführt werden kann. Als Öffnung zur Flüssigkeitszufuhr ist ein Durchlass innerhalb der mechanischen Struktur zu verstehen, durch den Reinigungs- oder Desinfektionsflüssigkeit auf das Wischtextil aufgebracht wird. Dadurch kann die Flüssigkeit gezielt zu bestimmten Kontaktpunkten zugeführt werden.

Die Anzahl der mechanischen Strukturen beträgt mindestens vier und ist geradzahlig, sodass sie gleichmäßig unter dem Wischtextil verteilt sind.

In einem Ausführungsbeispiel ist die Flüssigkeitszuführeinrichtung zur Bereitstellung und Zuführung von Wasser oder Reinigungsflüssigkeit vorgesehen, wobei zusätzlich eine Desinfektionsflüssigkeitszuführeinrichtung zur Bereitstellung und Zuführung von Desinfektionsflüssigkeit auf das Wischtextil vorgesehen ist. Mit anderen Worten weist die Reinigungs- und/oder Desinfektionsstation zwei Zuführeinrichtungen auf, wodurch das Reinigungs- und/oder Desinfektionssystem sowohl Reinigungsfunktionen als auch Desinfektionsfunktionen ausführen kann. Damit ist das Reinigungs- und/oder Desinfektionssystem flexibel einsetzbar.

Die Desinfektionsflüssigkeitszuführeinrichtung ist eine Vorrichtung zur Bereitstellung und Zuführung einer Desinfektionsflüssigkeit auf das Wischtextil. Sie kann als Einheit zur in-situ-Erzeugung einer Desinfektionsflüssigkeit ausgeführt sein, wobei bevorzugt Natriumhypochlorit als Desinfektionsflüssigkeit verwendet wird.

Ein vorgeschlagenes Reinigungs- und/oder Desinfektionssystem umfasst eine Reinigungs- und/oder Desinfektionsstation gemäß den obigen Ausführungen sowie ein Reinigungsgerät, das mit einem beweglichen, insbesondere rotierenden oder oszillierenden, Wischtextil ausgestattet ist. Es ist als modulares System ausgeführt und kann an unterschiedliche Reinigungsgeräte mit rotierenden oder oszillierenden Wischtextilien angepasst werden.

Ein Reinigungsgerät ist eine Vorrichtung zur Bodenreinigung, die ein Wischtextil zum Aufnehmen von Schmutz verwendet. Das Reinigungsgerät kann beispielsweise als Reinigungsroboter, insbesondere als Wischroboter, bevorzugt als Saug- und Wischroboter, ausgebildet sein. Reinigungsroboter kombinieren üblicherweise eine Staubsaugfunktion mit einer Nassreinigung und verfügen über mindestens ein Wischtextil, das während des Betriebs mit einer Reinigungsflüssigkeit befeuchtet wird, um Schmutz vom Boden aufzunehmen. Das Wischtextil kann dabei entweder stationär oder in Rotation bzw. Oszillation bewegt werden, um die Reinigungsleistung zu verbessern.

Die Reinigungs- und/oder Desinfektionsstation ist beispielsweise als Basisstation des Systems ausgebildet, zu der der Reinigungsroboter nach einem vordefinierten Reinigungsvorgang automatisch zurückkehrt. An der Basisstation erfolgt die Reinigung und/oder Desinfektion des Wischtextils, sodass der Reinigungsroboter im Anschluss an diesen Vorgang mit einem gesäuberten und/oder desinfizierten Wischtextil arbeiten kann. Die Basisstation kann dabei neben der Hygienisierung, also der Reinigung und/oder Desinfektion, auch weitere Funktionen wie das automatische Entleeren des Staubbehälters des Reinigungsroboters oder das Nachfüllen von Frischwasser ausführen. Durch die Integration der Reinigungs- und/oder Desinfektionsstation in die Basisstation wird ein vollständig automatisiertes Reinigungssystem geschaffen, das ohne manuelle Eingriffe eine kontinuierliche und effiziente Bodenreinigung ermöglicht.

Nicht beschränkende Ausführungsformen der Erfindung werden nun unter Bezug auf die beiliegenden Figuren genauer beschrieben, in denen:
- Figur 1: eine stark schematische Darstellung eines Reinigungs- und/oder Desinfektionssystems mit einer Reinigungs- und/oder Desinfektionsstation; und
- Figur 2: ein Blockschaltbild zur Veranschaulichung eines Verfahrens zur Reinigung und/oder Desinfektion eines Wischtextils eines Reinigungsgeräts in der Reinigungs- und/oder Desinfektionsstation nach Figur 1
darstellen.

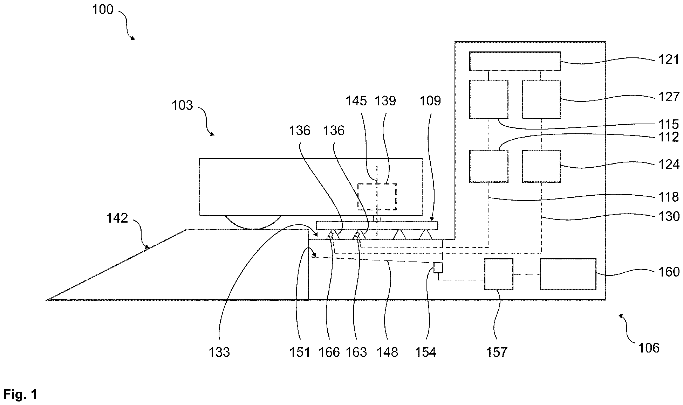

Das in Figur 1 dargestellte Reinigungs- und Desinfektionssystem 100 umfasst ein Reinigungsgerät 103 sowie eine Reinigungs- und Desinfektionsstation 106 zur Reinigung bzw. Desinfektion, also zum Hygienisieren, eines hier als kreisförmiges Wischtusch ausgebildeten Wischtextils 109 des Reinigungsgeräts 103. Die Reinigungs- und Desinfektionsstation 106 ist demnach als Hygienisierungsstation zur Hygienisierung des Wischtextils 109 eingerichtet und das Reinigungs- und Desinfektionssystem 100 ist ein Hygienisierungssystem. Das Reinigungsgerät 103 ist hier als Reinigungsroboter, bevorzugt als Wischroboter, ausgebildet. Der Reinigungsroboter kann zusätzlich eine Saugfunktion aufweisen. Das Reinigungsgerät 103 weist mindestens ein Wischtextil 109 auf, das während des normalen Reinigungsvorgangs einer Oberfläche Schmutz aufnimmt und anschließend in der Reinigungs- und/oder Desinfektionsstation 106 andockt und im vorliegenden Beispiel gereinigt und desinfiziert wird. Die Reinigungs- und/oder Desinfektionsstation 106 ist als Basisstation des Systems konzipiert.

Die Reinigungs- und Desinfektionsstation 106 umfasst eine - hier als Reinigungsflüssigkeitszuführeinrichtung eingerichtete - Flüssigkeitszuführeinrichtung 112, die dazu ausgebildet ist, eine definierte Menge Reinigungsflüssigkeit aus einem ersten Reservoir 115 und einer ersten Zuleitung 118 auf das Wischtextil 109 zu pulsieren. Die Reinigungs- und Desinfektionsstation 106 umfasst außerdem eine zweite Flüssigkeitszuführeinrichtung in Form einer Desinfektionsflüssigkeitszuführeinrichtung 124, die dazu ausgebildet ist, eine definierte Menge Desinfektionsflüssigkeit aus einem zweiten Reservoir 127 und einer zweiten Zuleitung 130 auf das Wischtextil 109 zu pulsieren. Die Flüssigkeitszuführeinrichtung 112 sowie die Desinfektionsflüssigkeitszuführeinrichtung 124 können jeweils als Pumpe, beispielsweise als Membran- oder Peristaltikpumpe, ausgeführt sein und sind über eine Steuerungseinheit 121 so steuerbar, dass die Flüssigkeitszufuhr in zeitlich getrennten Intervallen, also pulsierend, erfolgt.

Die Desinfektionsflüssigkeitszuführeinrichtung 124 ist hier eine in-situ-Erzeugungseinheit für Desinfektionsflüssigkeit. Als Desinfektionsflüssigkeit eignet sich insbesondere Natriumhypochlorit. Mittels der Reinigungs- und Desinfektionsstation 106 kann das Wischtextil 109 also nicht nur gereinigt, sondern auch desinfiziert werden.

Weiterhin umfasst die Reinigungs- und Desinfektionsstation 106 einen Abreinigungsbereich 133 zur Aufnahme des Wischtextils 109, in dem mechanische Strukturen 136 angeordnet sind. Diese mechanischen Strukturen 136 sind dazu ausgelegt, das Wischtextil 109 mechanisch auszupressen, indem das Wischtextil 109 über die Strukturen 136 bewegt wird. Hierzu weist das Reinigungsgerät 103 einen Aktuator 139 auf, welcher grundsätzlich dazu eingerichtet ist, das Wischtextil 109 im normalen Reinigungsvorgang, bei dem ein befahrener Untergrund 142 mit dem Wischtextil 109 gewischt wird, um eine Rotationsachse 145 zu rotieren oder zu oszillieren. Diese Funktion wird in einem hierin beschriebenen Reinigungs- bzw. Desinfektionsverfahren genutzt, um das Wischtextil 109 mittels der mechanischen Strukturen 136 im Abreinigungsbereich 133 zu reinigen bzw. zu desinfizieren.

Die mechanischen Strukturen 136 sind hier als in Richtung des Wischtextils 109 spitz zulaufende Vorsprünge mit einer im Wesentlichen dreieckigen Querschnittsform ausgebildet, wobei die Flanken dieser Vorsprünge eine aus dem Wischtextil 109 ausgepresste Flüssigkeit gezielt in eine Auffangeinrichtung 148 der Reinigungs- und Desinfektionsstation 106 leiten.

Die Auffangeinrichtung 148 ist als wasserdichte, vorzugsweise am Grund 151 geneigte Wanne ausgeführt und weist einen Ablauf 154 auf, wobei eine abgeleitete und in der Auffangeinrichtung 148 aufgefangene Flüssigkeit in den an der tiefsten Stelle der Wanne angeordneten Ablauf 154 geführt und gesammelt wird. Über eine Flüssigkeitsabführeinrichtung 157, die beispielsweise als Pumpe oder Unterdrucksystem ausgeführt sein kann, wird die Schmutzflüssigkeit in einen Abwassersammelbehälter 160 befördert. Der Abwassersammelbehälter 160 ist als geschlossenes System ausgebildet, um das Entweichen von Flüssigkeitsdämpfen zu verhindern. Zusätzlich kann der Abwassersammelbehälter 160 mit einem Aktivkohlefilter oder einem Kondensationssystem versehen sein, um flüchtige Substanzen zu binden und eine Geruchsbildung zu reduzieren.

Nach Figur 1 weisen zwei der mechanischen Strukturen 136 jeweils eine Öffnung 163, 166 auf, wobei die eine der beiden Öffnungen 163 den Abreinigungsbereich 133 mit der ersten Zuleitung 118 fluidisch verbindet, und wobei die andere der beiden Öffnungen 166 den Abreinigungsbereich 133 mit der zweiten Zuleitung 130 fluidisch verbindet. Über die erste Öffnung 163 wird Reinigungsflüssigkeit auf das Wischtextil 109 aufgebracht, wobei über die zweite Öffnung 166 Desinfektionsflüssigkeit auf das Wischtextil 109 aufgebracht wird.

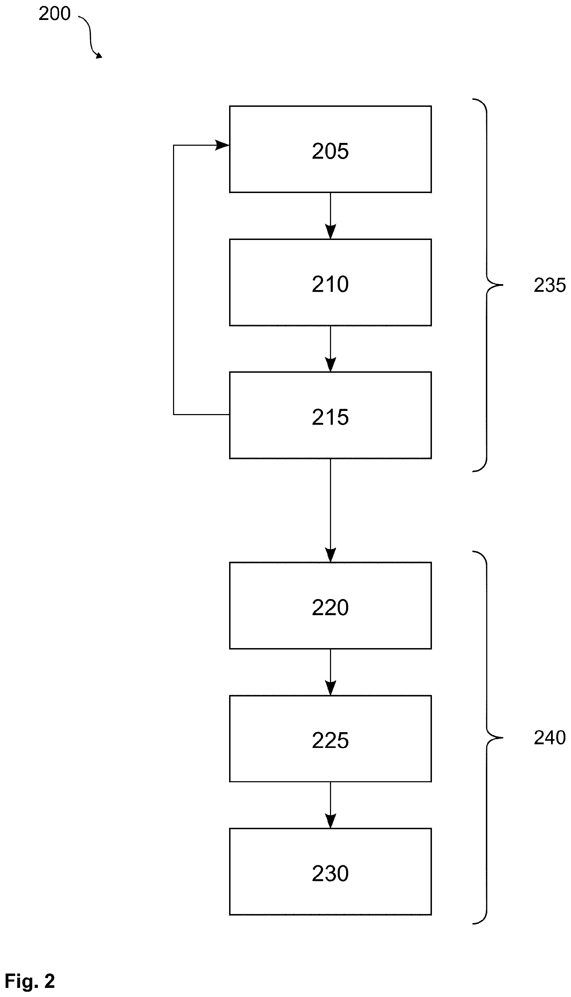

Mit Figur 2 soll mit einem Blockschaltbild ein Verfahren 200 zur Reinigung bzw. Desinfektion des Wischtextils 109 des Reinigungsgeräts 103 in der Reinigungs- und Desinfektionsstation 106 nach Figur 1 veranschaulicht werden.

Das Verfahren 200 wird beispielhaft anhand eines Reinigungsvorgangs des Wischtextils 109 mit daran anschließendem Desinfektionsvorgang beschrieben. Nachdem das Reinigungsgerät 103, hier also der Reinigungsroboter, an der Reinigungs- und Desinfektionsstation 106 angedockt ist und sich das Wischtextil 109 im Abreinigungsbereich 133 befindet, beginnt das Verfahren 200 in einem ersten Schritt 205 mit einem pulsierenden Zuführen einer definierten Menge einer Reinigungsflüssigkeit aus dem ersten Reservoir 115 und über die erste Zuleitung 118 auf das Wischtextil 109 durch die Flüssigkeitszuführeinrichtung 112 für eine vorgegebene erste Zeitdauer. Anschließend wird das Wischtextil 109 in einem zweiten Schritt 210 über die mechanischen Strukturen 136 bewegt, indem das Wischtextil 109 mittels des Aktuators 139 beispielsweise um die Rotationsachse 145 rotiert wird. Schmutz und überschüssige Flüssigkeit wird von den Strukturen 136 aus dem Wischtextil 109 ausgepresst und in die Auffangeinrichtung 148 geleitet, wo die Flüssigkeit gesammelt wird.

In einem dritten Schritt 215 wird die Flüssigkeitszufuhr gestoppt, während die Bewegung des Wischtextils 109 über die mechanischen Strukturen 136 für eine vorgegebene zweite Zeitdauer fortgesetzt wird, um eine maximale Entwässerung des Wischtextils 109 zu erzielen. Die zweite Zeitdauer ist dabei mindestens fünfmal länger als die erste Zeitdauer.

Während der Schritte 205, 210 und 215 wird die in der Auffangeinrichtung 148 gesammelte Flüssigkeit mittels der Flüssigkeitsabführeinrichtung 157 über den Ablauf 154 in den Abwassersammelbehälter 160 abgeleitet. Optional kann der Verschmutzungsgrad der abgeleiteten Flüssigkeit überwacht werden.

In Abhängigkeit eines Verschmutzungsgrades der abgeleiteten bzw. abgesaugten Flüssigkeit können die Schritte 205 bis 215 wiederholt werden kann, bis der Verschmutzungsgrad der Flüssigkeit unter einen vorgegebenen Grenzwert fällt. Eine - hier nicht gezeigte - Sensorik kann dazu eingerichtet sein, den Verschmutzungsgrad der abgeleiteten bzw. abgesaugten Flüssigkeit zu erfassen. Die Steuerungseinheit 121 kann das System 100 anhand der erfassten Daten steuern.

Durch die Kombination aus pulsierender Flüssigkeitszufuhr, mechanischer Auspressung und kontrollierter Flüssigkeitsableitung wird eine besonders effiziente und flüssigkeitssparende Desinfektion des Wischtextils 109 erzielt.

Nach Abschluss des Reinigungsvorgangs kann in einem weiteren Schritt 220 mit dem pulsierenden Zuführen einer definierten Menge Desinfektionsflüssigkeit aus dem zweiten Reservoir 127 auf das Wischtextil 109 durch die Desinfektionsflüssigkeitszuführeinrichtung 124 für eine vorgegebene erste Zeitdauer. Anschließend wird das Wischtextil 109 in einem darauffolgenden Schritt 225 über die mechanischen Strukturen 136 bewegt, analog zu Schritt 210. Überschüssige Desinfektionsflüssigkeit wird von den Strukturen 136 aus dem Wischtextil 109 ausgepresst und in die Auffangeinrichtung 148 geleitet, gesammelt und analog zu den obigen Ausführungen in den Abwassersammelbehälter 160 abgesaugt. "Abwasser" ist in der vorliegenden Erfindung als Überbegriff für die aus dem Reinigungs- und Desinfektionsvorgang überschüssige und oftmals verschmutzte Flüssigkeit, die im als geschlossener Tank ausgeführten Abwassersammelbehälter 160 gesammelt und in regelmäßigen Abständen, insbesondere wenn der Behälter voll ist, entleert werden kann.

In einem weiteren Schritt 230 wird die Flüssigkeitszufuhr gestoppt, während die Bewegung des Wischtextils 109 über die mechanischen Strukturen 136 für eine vorgegebene zweite Zeitdauer fortgesetzt wird, um eine maximale Entwässerung des Wischtextils 109 zu erzielen. Die zweite Zeitdauer ist dabei mindestens fünfmal länger als die erste Zeitdauer.

Durch die Kombination aus pulsierender Flüssigkeitszufuhr, mechanischer Auspressung und kontrollierter Flüssigkeitsableitung wird eine besonders effiziente und wassersparende Reinigung des Wischtextils 109 erzielt.

Das hierin beschriebene Verfahren hat den Vorteil, dass die verwendete Flüssigkeitsmenge zum Abreinigen des Wischtextils 109 in der Station 106 reduziert wird. Ebenso kann die erforderliche Menge Desinfektionsflüssigkeit deutlich reduziert werden. Infolge des hinsichtlich des Volumens geringeren Eintrags an Desinfektionsflüssigkeit in das Wischtextil 109 sinkt folglich das Risiko einer Grenzwertüberschreitung eines unerwünschten und ggfs. gesundheitsschädlichen Gases deutlich. Um dieses Risiko weiter zu reduzieren, kann die Flüssigkeitsabführeinrichtung 157 nach dem Desinfektionsvorgang für eine bestimmte Zeitdauer nachlaufen.

Nach den Schritt 230 kann mit dem Reinigungsgerät 103 eine übliche Reinigungsfunktion ausgeführt werden. Wenn die Reinigungsfunktion abgeschlossen ist, kann das Verfahren 200 erneut ausgeführt werden.

Denkbar ist, dass das System 100 ausschließlich als Reinigungssystem zur Reinigung des Wischtextils 109 ausgebildet ist, sodass die Station 106 als Reinigungsstation ausgeführt ist. In diesem Fall kann nur ein erster Teil 235 des Verfahrens ausgeführt werden, der die Schritte 205 bis 215 umfasst.

Das System 100 kann alternativ nur als Desinfektionssystem zur Desinfektion des Wischtextils 109 ausgebildet sein, sodass die Station 106 als Desinfektionsstation ausgeführt ist. In diesem Fall wird nur ein zweiter Teil 240 des Verfahrens 200 ausgeführt, welcher die Schritte 220 bis 230 umfasst.

### Bezugszeichen

- 100: Reinigungs- und/oder Desinfektionssystem
- 103: Reinigungsgerät
- 106: Reinigungs- und/oder Desinfektionsstation
- 109: Wischtextil
- 112: Flüssigkeitszuführeinrichtung
- 115: Erstes Reservoir
- 118: Erste Zuleitung
- 121: Steuerungseinheit
- 124: Desinfektionsflüssigkeitszuführeinrichtung
- 127: Zweites Reservoir
- 130: Zweite Zuleitung
- 133: Abreinigungsbereich
- 136: Mechanische Struktur
- 139: Aktuator
- 142: Untergrund
- 145: Rotationsachse
- 148: Auffangeinrichtung
- 151: Grund der Auffangeinrichtung
- 154: Ablauf
- 157: Flüssigkeitsabführeinrichtung
- 160: Abwassersammelbehälter
- 163: Öffnung
- 166: Öffnung

- 200: Verfahren
- 205: Reinigungsflüssigkeit zuführen
- 210: Wischtextil bewegen
- 215: Zufuhr stoppen
- 220: Desinfektionsflüssigkeit zuführen
- 225: Wischtextil bewegen
- 230: Zufuhr stoppen
- 235: erster Teil
- 240: zweiter Teil

## Patentansprüche

1. Verfahren (200) zur Reinigung und/oder Desinfektion eines Wischtextils (109) eines Reinigungsgeräts (103) in einer Reinigungs- und/oder Desinfektionsstation (106), umfassend die Schritte:
a) Pulsierendes Zuführen einer definierten Menge einer Reinigungs- oder Desinfektionsflüssigkeit auf das Wischtextil (109) für eine vorgegebene erste Zeitdauer,
b) Bewegen des Wischtextils (109) über mechanische Strukturen (136), um Schmutz sowie überschüssige Flüssigkeit aus dem Wischtextil (109) auszupressen und in eine Auffangeinrichtung (148) abzuleiten, und
c) Stoppen der Flüssigkeitszufuhr und Fortsetzen der Bewegung des Wischtextils (109) über die mechanischen Strukturen (136) für eine vorgegebene zweite Zeitdauer, wobei die erste Zeitdauer jeweils kürzer ist als die nachfolgende zweite Zeitdauer.

2. Verfahren (200) nach Anspruch 1, wobei während Schritt a), Schritt b) und/oder Schritt c) mittels der der Auffangeinrichtung (148) aufgefangene Flüssigkeit über einen Ablauf (154) in einen Abwassersammelbehälter (160) abgeleitet wird.

3. Verfahren (200) nach Anspruch 1 oder Anspruch 2, wobei die zweite Zeitdauer mindestens fünfmal länger ist als die erste Zeitdauer.

4. Verfahren (200) nach einem der vorangehenden Ansprüche, wobei ein Verschmutzungsgrad der in den Abwassersammelbehälter (160) abgeleiteten Flüssigkeit erfasst wird.

5. Verfahren (200) nach Anspruch 4, wobei die Schritte a) bis c) wiederholt werden, bis der erfasste Verschmutzungsgrad einen Grenzwert unterschreitet.

6. Verfahren (200) nach einem der vorhergehenden Ansprüche, wobei das Wischtextil (109) während Schritt b) und/oder Schritt c) rotierend oder oszillierend bewegt wird.

7. Reinigungs- und/oder Desinfektionsstation (106) für ein Reinigungs- und/oder Desinfektionssystem (100) zur Reinigung und/oder Desinfektion eines Wischtextils (109) eines Reinigungsgeräts (103), umfassend:
- eine Flüssigkeitszuführeinrichtung (112) zum pulsierenden Zuführen einer definierten Menge Reinigungs- oder Desinfektionsflüssigkeit auf das Wischtextil (109),
- eine Auffangeinrichtung (148) zum Auffangen von Flüssigkeit, und
- einen Abreinigungsbereich (133) mit mechanischen Strukturen (136), die dazu ausgebildet sind, durch mechanische Bearbeitung des Wischtextils (109) Schmutz sowie überschüssige Flüssigkeit auszupressen und in die Auffangeinrichtung (148) abzuleiten.

8. Reinigungs- und/oder Desinfektionsstation (106) nach Anspruch 7, wobei die Auffangeinrichtung (148) einen Ablauf (154) zum Ableiten der aufgefangenen Flüssigkeit in einen Abwassersammelbehälter (160) aufweist.

9. Reinigungs- und/oder Desinfektionsstation (106) nach Anspruch 8, wobei eine Flüssigkeitsabführeinrichtung (157) vorgesehen und dazu ausgebildet ist, in der Auffangeinrichtung (148) gesammelte Flüssigkeit über den Ablauf (154) in den Abwassersammelbehälter (160) abzuführen.

10. Reinigungs- und/oder Desinfektionsstation (106) nach Anspruch 8 oder Anspruch 9, wobei der Abwassersammelbehälter (160) als geschlossenes System ausgebildet ist, um das Entweichen von Flüssigkeitsdämpfen zu verhindern.

11. Reinigungs- und/oder Desinfektionsstation (106) nach einem der Ansprüche 7 bis 10, wobei die mechanischen Strukturen (136) als Vorsprünge mit einer im Wesentlichen dreieckigen Querschnittsform ausgebildet sind, und wobei die Vorsprünge mit dem Wischtextil (109) in Kontakt kommen.

12. Reinigungs- und/oder Desinfektionsstation (106) nach einem der Ansprüche 7 bis 11, wobei mindestens eine der mechanischen Strukturen (136) eine oder mehrere Öffnungen (163) zur Flüssigkeitszufuhr aufweist.

13. Reinigungs- und/oder Desinfektionsstation (106) nach einem der Ansprüche 7 bis 12, wobei die Anzahl der mechanischen Strukturen (136) mindestens vier beträgt und geradzahlig ist.

14. Reinigungs- und/oder Desinfektionsstation (106) nach einem der Ansprüche 7 bis 13, wobei die Flüssigkeitszuführeinrichtung (112) zur Bereitstellung und Zuführung von Wasser oder Reinigungsflüssigkeit vorgesehen ist, und wobei eine Desinfektionsflüssigkeitszuführeinrichtung (124) zur Bereitstellung und Zuführung von Desinfektionsflüssigkeit auf das Wischtextil (109) vorgesehen ist.

15. Reinigungs- und/oder Desinfektionssystem (100), umfassend eine Reinigungs- und/oder Desinfektionsstation (106) nach einem der Ansprüche 7 bis 14 sowie ein Reinigungsgerät (103), das wenigstens ein bewegliches, insbesondere rotierendes oder oszillierendes, Wischtextil (109) aufweist.
